# EUROPÄISCHE PATENTANMELDUNG

(11) **EP 1 275 347 A1**
(43) Veröffentlichungstag der Anmeldung: **15.01.2003**
(21) Anmeldenummer: 01116869.7
(22) Anmeldetag: 10.07.2001
(51) Int. Cl.: A61B 17/70

(54) **Pedikelschraubenanordnung**

(71) Anmelder: Waldemar Link (GmbH & Co.), 22339 Hamburg (DE)
(72) Erfinder: Link, Helmut D., 22397 Hamburg (DE)
(74) Vertreter: Glawe, Delfs, Moll & Partner

(57) **Zusammenfassung**

Pedikelschraubenanordnung mit einer Pedikelschraubenstütze (4). Diese weist eine mit der Pedikelschraube (1) zusammenwirkende Stützfläche (5) und eine oder mehrere Stützflächen (6, 7) auf, die sich an einer geeigneten Knochenfläche abstützen, insbesondere an der Fläche (3) des zwischen dem processus costalis und dem processus articularis superior (2) befindlichen Sattels.

## Beschreibung

Zur Stabilisierung, Ausrichtung oder Fusion von Wirbelkörpern verwendet man Wirbelsäuleninstrumentarien, die aus Pedikelschrauben und diese verbindenden Stangen bestehen. Die Pedikelschrauben werden zwischen dem processus costalis und dem processus articularis superior durch den Pedikel in den Wirbelkörper geschraubt. Der Pedikel ist äußerst kräftig und vermag die von den Pedikelschrauben übertragenen Kräfte aufzunehmen. Mitunter kommt es aufgrund der hohen Kräfte und insbesondere Biegemomente, die auf die Pedikelschrauben wirken, zu Schraubenbrüchen.

Der Erfindung liegt die Aufgabe zugrunde, diesen Nachteil zu vermindern.

Die erfindungsgemäße Lösung liegt in den Merkmalen des Anspruchs 1. Vorteilhafte Weiterbildungen ergeben sich aus den Unteransprüchen.

Der Gefahr von Schraubenbrüchen versucht die Erfindung vorzubeugen, indem sie eine Pedikelschraubenstütze vorsieht, die einerseits eine Stützfläche oder Stützflächen für den aus dem Knochen frei herausragenden Schraubenschaft bildet und andererseits eine oder mehrere Stützflächen aufweist, die sich an einer geeigneten Stelle des Knochens abstützt.

Da die Pedikelschraube durch die Sattelfläche zwischen dem processus articularis superior und dem processus costali in den Knochen eintritt, ist es zweckmäßig, eine oder mehrere solcher Stützflächen zur Anlage an dieser Sattelfläche vorzusehen, und zwar vorzugsweise oberhalb und unterhalb der Schraubeneintrittsstelle. Da hauptsächlich vertikale Kräfte zu übertragen sind, sollten diese Stützflächen eine starke Querkomponente zu demjenigen Teil der Sattelfläche aufweisen, durch die die Schraube eintritt. Oder, anders ausgedrückt, sollen sie möglichst weitgehend parallel zur Schraubenrichtung verlaufen, vorzugsweise in einem Winkel von weniger als 40°. Das ist auch die Richtung des Pedikels (pediculus arcus vertebrae).

Ein anderer geeigneter Ort zur Abstützung ist die Unterseite und/oder Oberseite des processus costali. Ebenso kann sich die Pedikelschraubenstütze an der Unterseite des processus articularis superior abstützen. Auch eine oberseitige Abstützung am processus articularis superior ist denkbar.

Ausführungsbeispiele werden anhand der Zeichnung erläutert. Darin zeigen:
- Fig. 1: einen Sagittalschnitt durch die Pedikelschraubeneintrittsstelle einer ersten Ausführungsform,
- Fig. 2: eine Dorsalansicht in Richtung der Pedikelschraubenachse und Fig. 9 eine Ansicht dieser Region von oben;
- Fig. 4-6: zeigen entsprechende Darstellungen einer zweiten und
- Fig. 7-9: entsprechende Darstellungen einer dritten Ausführungsform.

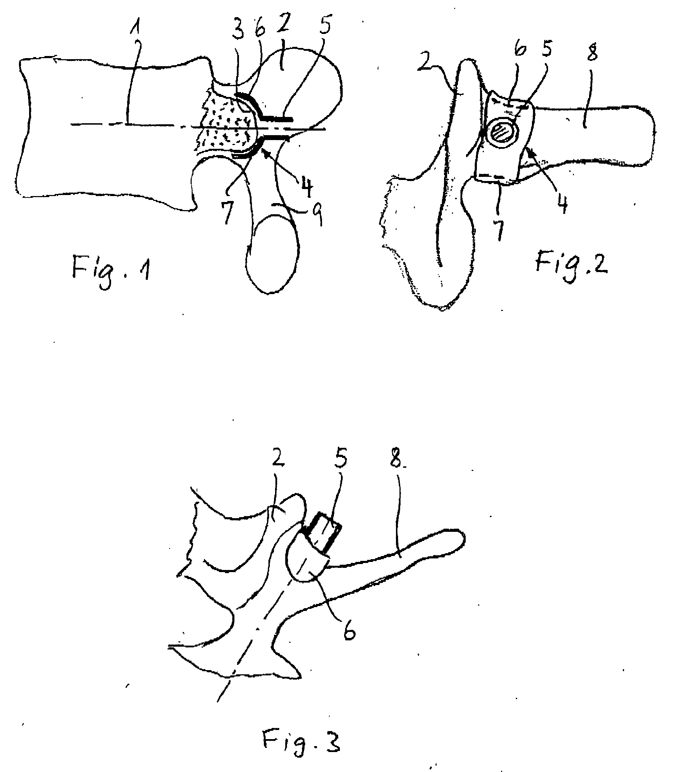

In Fig. 1 ist strichpunktiert die Achse 1 einer Pedikelschraube dargestellt, die zwischen dem processus articularis superior 2 und dem vor der Zeichenebene liegenden processus costali in die Oberfläche des Sattels 3 zwischen diesen beiden Fortsätzen eintritt. Die Pedikelschraubenstütze 4 weist einen rohrförmigen Stützteil 5 für den Schraubenschaft auf. Eine hinreichend enge Passung sorgt dafür, daß die Stütze 4 an etwaigen Bewegungen des Schraubenschafts teilnehmen muß oder - umgekehrt ausgedrückt - , daß sie den Schraubenschaft stützt, insoweit sie dessen Bewegungstendenzen nicht folgen will.

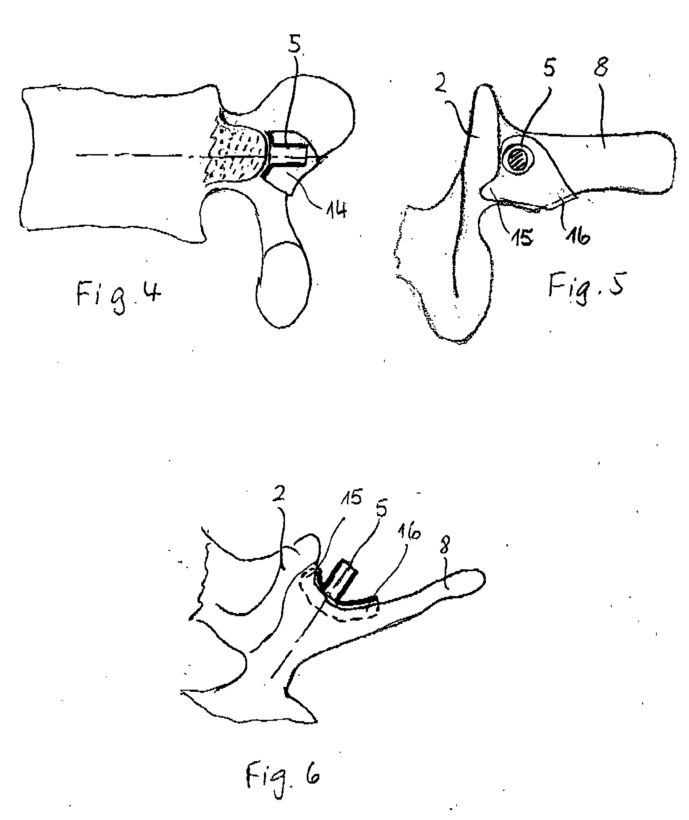

Die Stütze 4 weist passend zu der Sattelfläche 3 gebogene Flächen oder Ausleger 6 und 7 auf, die sich an die Sattel-oberfläche 3 weitgehend anlegen und diese auch so weit umfassen, daß ihre Endbereiche sich der Richtung des Schraubenschafts bis auf weniger als 20°, vorzugsweise weniger als 10°, annähern. Zusätzlich oder statt dessen können die Flächenteile 6, 7 mit Zähnen oder Schrauben versehen werden, die in die Sattelfläche 3 eindringen, um der Schraubenstütze 4 zusätzliche Stabilität am Knochen zu verleihen. Besonders deutlich sieht man in Fig. 2, wie die Flügel 6, 7 der Stütze 4 sich mit ihren Enden über und unter die Sattelfläche legen, die zwischen dem processus articularis superior 2 und dem processus costali 8 liegt. Dasselbe geht aus der Draufsicht in Fig. 3 bezüglich des Flügels 6 hervor.

Das zweite Ausführungsbeispiel gemäß Fig. 5 zeigt, daß das Stützrohr 5, das den Schraubenschaft eng umgeben soll, mit einem geformten Blechteil 14 starr verbunden ist, das mit einem ersten Flügel 15 unter den processus articularis superior und mit einem zweiten Flügel 16 unter den processus costali greift. Kräfte, die zur Anhebung des Schraubenschafts tendieren, werden dadurch sicher auf die genannten Knochenflächen übertragen.

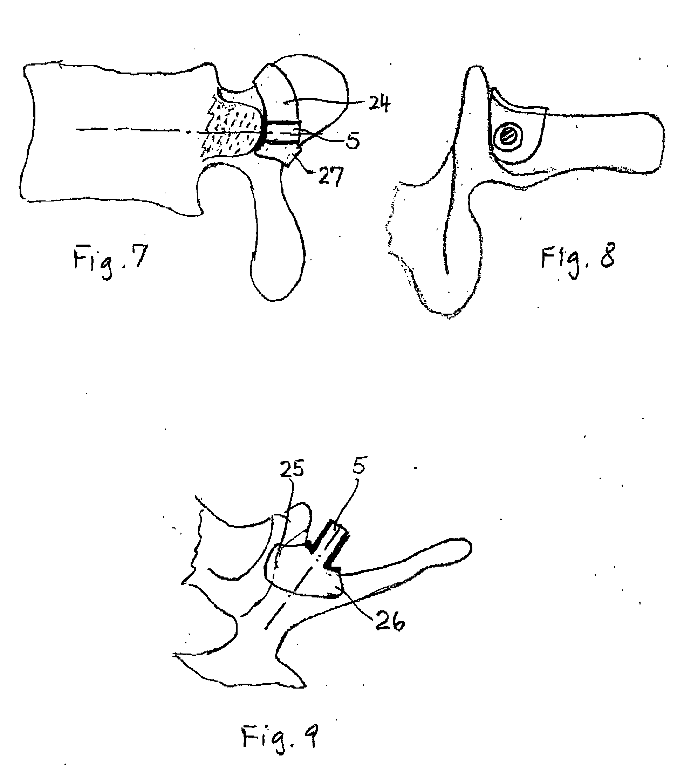

Das Ausführungsbeispiel gemäß Fig. 7 bis 9 zeigt Entsprechendes für eine oberseitige Abstützung. Das den Schraubenschaft aufnehmende Rohr 5 ist: starr mit einem Blechteil 24 verbunden, der mit einem ersten Flügel 25 über den processus articularis superior und mit einem zweiten Flügel 26 über den processus costali greift. Es kann ein weiterer Flügel 27 vorhanden sein, der unter den processus articularis superior und/oder unter den processus costali greift. Die Ausführungsformen gemäß Fig. 4 bis 6 einerseits und Fig. 7 bis 9 andererseits können somit in einem Formstück vereinigt sein.

Die Knochenformen in dem betreffenden Bereich sind äußerst vielgestaltig. Zweckmäßig ist es deshalb, eine mehr oder weniger große Anzahl von Formstücken je nach Anwendungserfordernis bereitzustellen. Es besteht auch die Möglichkeit, die Pedikelschraubenstütze aus mehreren Teilen, die zur Abstützung an unterschiedlicher Stelle geeignet sind, mittels entsprechender Verbindungseinrichtungen an Ort und Stelle starr zusammenzufügen oder wenigstens mit dem Schraubenschaft zu verbinden.

Auch eine Abstützung an dem processus articularis inferior, der in Fig. 1 mit der Bezugsziffer 9 bezeichnet ist, kann im Rahmen der Erfindung stattfinden.

## Patentansprüche

1. Pedikelschraubenanordnung, **dadurch gekennzeichnet, daß** eine Pedikelschraubenstütze (4, 14, 24) vorgesehen ist, die einerseits mindestens eine erste, mit der Pedikelschraube (1) zusammenwirkende Stützfläche (5) und andererseits mindestens eine zweite, zur Anlage an dem processus costalis (8) und/oder dem processus articularis superior (2) und/oder an der zwischen diesen befindlichen Sattelfläche (3) und/oder an dem processus articularis inferior ausgebildete Stützfläche (6, 7) aufweist.

2. Pedikelschraubenanordnung nach Anspruch 1, **dadurch gekennzeichnet, daß** die mit der Pedikelschraube zusammenwirkende Stützfläche (5) eine Bohrung ist.

3. Pedikelschraubenanordnung nach Anspruch 2, **dadurch gekennzeichnet, daß** die Bohrung ein langgestreckter Zylinder ist.

4. Pedikelschraubenanordnung nach einem der Ansprüche 1 bis 3, **dadurch gekennzeichnet, daß** die zweite Stützfläche ein unter den processus costalis (8) und/oder den processus articularis superior (2) greifenden Flächenanteil (15, 16) aufweist.

5. Pedikelschraubenanordnung nach einem der Ansprüche 1 bis 3, **dadurch gekennzeichnet, daß** die zweite Stützfläche unter den zwischen processus articularis superior (2) und processus costalis (8) befindlichen Sattel (3) greift.

6. Pedikelschraubenanordnung nach einem der Ansprüche 1 bis 4, **dadurch gekennzeichnet, daß** die zweite Stützfläche über den zwischen dem processus articularis superior (2) und processus costalis (8) befindlichen Sattel (3) greift.

7. Pedikelschraubenanordnung nach einem der Ansprüche 1 bis 6, **dadurch gekennzeichnet, daß** die Pedikelschraubenstütze mit Zähnen oder Schrauben zur Verbesserung ihres Sitzes an der Knochenoberfläche ausgerüstet ist.
